(19) Europäisches Patentamt / European Patent Office / Office européen des brevets

(11) EP 3 191 189 B1

(12) EUROPÄISCHE PATENTSCHRIFT

(45) Veröffentlichungstag und Bekanntmachung des Hinweises auf die Patenterteilung: **02.10.2019 Patentblatt 2019/40**

(21) Anmeldenummer: **15750753.4**

(22) Anmeldetag: **18.08.2015**

(51) Int Cl.: ***A61Q 5/10*** *(2006.01)* ***A61K 8/898*** *(2006.01)*

(86) Internationale Anmeldenummer: **PCT/EP2015/068876**

(87) Internationale Veröffentlichungsnummer: **WO 2016/037803 (17.03.2016 Gazette 2016/11)**

(54) **VERPACKUNGSEINHEIT (KIT-OF-PARTS) MIT SPEZIELLEN AMINIERTEN SILICONPOLYMEREN**

PACKAGING UNIT (KIT-OF-PARTS) HAVING PARTICULAR AMINATED SILICONE POLYMERS

UNITÉ DE CONDITIONNEMENT (KIT-OF-PART) COMPRENANT DES POLYMÈRES DE SILICONE AMINÉS SPÉCIFIQUES

(84) Benannte Vertragsstaaten: **AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.09.2014 DE 102014218006**

(43) Veröffentlichungstag der Anmeldung: **19.07.2017 Patentblatt 2017/29**

(73) Patentinhaber: **Henkel AG & Co. KGaA 40589 Düsseldorf (DE)**

(72) Erfinder:
- **KERL, Sylvia 22763 Hamburg (DE)**
- **BIETZ, Susanne 25336 Elmshorn (DE)**

(56) Entgegenhaltungen:
**EP-A1- 1 426 038**
**WO-A2-02/47632**
**WO-A2-03/009822**
**WO-A2-2012/079915**
**FR-A1- 2 945 731**
**US-A1- 2003 152 534**
**US-A1- 2003 229 947**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 191 189 B1

## Beschreibung

Die vorliegende Erfindung betrifft Erfindung eine Verpackungseinheit (Kit-of-parts), enthaltend ein kosmetisches Mittel mit speziellen aminierten Siliconpolymeren sowie eine Oxidationsmittelzubereitung.

Weiterhin betrifft die vorliegende Erfindung ein Verfahren zum Färben von keratinischen Fasern unter Verwendung einer erfindungsgemäßen Verpackungseinheit.

Schließlich betrifft die vorliegende Erfindung die Verwendung einer erfindungsgemäßen Verpackungseinheit zur Herstellung eines kosmetischen Mittels zur Farbveränderung keratinischer Fasern mit erhöhter Pflege der keratinischer Fasern und/oder verbesserter Buntheit von Farbnuancen.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Im Stand der Technik werden Verpackungseinheiten zur Färbung keratinischer Fasern offenbart, welche getrennt konfektioniert a) ein kosmetisches Mittel, welches Oxidationsfarbstoffvorprodukte und/oder direktziehende Farbstoffe und ein Silikon sowie b) eine Oxidationsmittelzubereitung, enthaltend 0,5 bis 15 Gew.-% Oxidationsmittel enthalten. Beispielhaft sind hier die US 2003/152534 A1, die WO 03/009822 A2, die US 2003/229947 A1, die WO 02/47632 A2, die FR 2 945 731 A1, die EP 1 426 038 A1 und die WO 2012/079915 A2 zu nennen.

Die im Stand der Technik bekannten Färbemittel führen jedoch nicht immer zu der gewünschten hohen Färbeleistung oder weisen keine ausreichenden zusätzlichen gewünschten Eigenschaften, wie beispielsweise eine verbesserte Pflege der Haare während der Haarfärbung, auf.

Der vorliegenden Erfindung lag daher die Aufgabe zugrunde, ein kosmetisches Mittel zur Färbung keratinischer Fasern bereitzustellen, welches die Nachteile des Standes der Technik vermeidet bzw. zumindest abschwächt und welches in einer verbesserten Pflege der Haare und/oder einer verbesserten Buntheit von Farbnuancen, d. h. in einem erhöhten Chroma-Wert, resultiert.

Es wurde nun überraschenderweise gefunden, dass der Einsatz von mindestens einem speziellen aminierten Siliconpolymer in kosmetischen Mitteln, welche einen Bestandteil einer Verpackungseinheit (Kit-of-parts) darstellen, zu einer verbesserten Pflege keratinischer Fasern und/oder einer verbesserten Buntheit von Farbnuancen führt. Ein Indikator für die verbesserte Pflege ist eine erhöhte Kämmbarkeit, insbesondere Nasskämmbarkeit, wohingegen ein Indikator für eine verbesserte Buntheit von Farbnuancen ein höherer Chromawert ist. Durch den erfindungsgemäßen Einsatz

mindestens eines speziellen aminierten Siliconpolymers werden ein höherer Chromawert und/oder eine verbesserte Nasskämmbarkeit erhalten.

Ein erster Gegenstand der Erfindung ist daher eine Verpackungseinheit (Kit-of-Parts) zur Färbung keratinischer Fasern, umfassend - getrennt voneinander konfektioniert -

a) mindestens einen Container (C1), enthaltend ein kosmetisches Mittel (M1), welches mindestens eine Verbindung, ausgewählt aus der Gruppe von Oxidationsfarbstoffvorprodukten, direktziehenden Farbstoffen sowie deren Mischungen, und mindestens ein aminiertes Siliconpolymer, enthaltend mindestens eine Struktureinheit der Formel (I) und mindestens eine Struktureinheit der Formel (II)

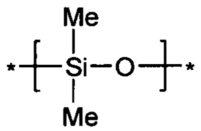
(I)
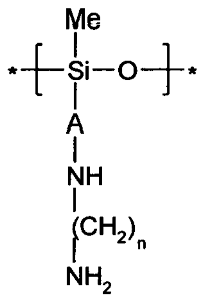
(II),

worin

A für eine lineare oder verzweigte $C_4$-$C_8$-Alkylgruppe steht, und
n für ganze Zahlen von 1 bis 4 steht, enthält,
wobei das kosmetische Mittel (M1) in dem Container (C1) als aminiertes Siliconpolymer vorliegt, enthaltend mindestens eine Struktureinheit der Formel (I) und mindestens eine Struktureinheit der Formel (II), darunter mindestens ein aminiertes Siliconpolymer der Formel (III) in einer Gesamtmenge von 0,05 bis 1,0 Gew.-%, bezogen auf das Gesamtgewicht des kosmetischen Mittels (M1), enthält

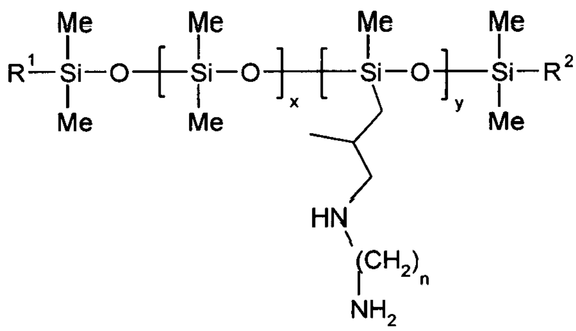
(III),

worin

$R^1$ und $R^2$, jeweils unabhängig voneinander, für eine Methylgruppe stehen,
x für ganze Zahlen von 49 bis 149, steht,
y für ganze Zahlen von 1 bis 10, steht, und
n für ganze Zahlen insbesondere 2 oder 3, steht,
wobei das kosmetische Mittel (M1) zusätzlich ein cyclisches aminiertes Siliconpolymer in einer Gesamtmenge von 0,015 bis 0,3 Gew.-%, bezogen auf das Gesamtgewicht des kosmetischen Mittels (M1), enthält, wobei das cyclische aminierte Siliconpolymer die Formel (IV) aufweist

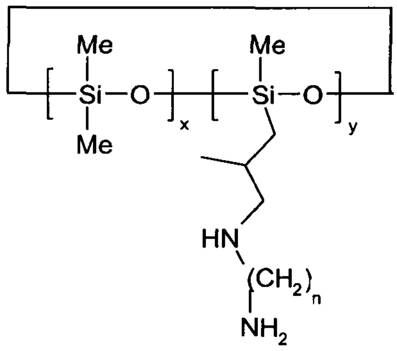

(IV),

worin

x für ganze Zahlen von 49 bis 149, steht, und
y für ganze Zahlen von 1 bis 10, steht

b) mindestens einen Container (C2), enthaltend eine Oxidationsmittelzubereitung (M2), welche in einem kosmetisch verträglichen Träger mindestens ein Oxidationsmittel in einer Gesamtmenge von 0,5 bis 15 Gew.-%, vorzugsweise von 0,75 bis 10 Gew.-%, bevorzugt von 1 bis 7,5 Gew.-%, besonders bevorzugt von 1,25 bis 7 Gew.-%, insbesondere von 1,5 bis 6,0 Gew.-%, bezogen auf das Gesamtgewicht der Oxidationsmittelzubereitung, enthält.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin wird unter dem Begriff "Container" im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Zudem werden unter dem Begriff "aminierte Siliconpolymere" im Rahmen der vorliegenden Erfindung Siliconpolymere verstanden, welche mindestens eine Aminogruppe pro Siliconpolymer aufweisen.

Darüber hinaus wird unter dem Begriff "Kämmbarkeit" im Rahmen der vorliegenden Erfindung sowohl die Kämmbarkeit der nassen Faser, als auch die Kämmbarkeit der trockenen Faser verstanden.

Weiterhin werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels (M1) bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels-sofern nichts anderes angegeben ist-auf das Gesamtgewicht des oxidationsmittelfreien erfindungsgemäßen kosmetischen Mittels.

Die kosmetischen Mittel (M1) in dem Container (C1) enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung enthält mindestens 30 Gew.-%, insbesondere mindestens 50 Gew.-%, Wasser, bezogen auf das Gesamtgewicht des kosmetischen Mittels (M1).

Unter wässrig-alkoholischen Trägern sind im Sinne der vorliegenden Erfindung wasserhaltige Zusammensetzungen, enthaltend einen $C_1$-$C_4$-Alkohol in einer Gesamtmenge von 3 bis 90 Gew.-%, bezogen auf das Gesamtgewicht des kosmetischen Mittels (M1), insbesondere Ethanol bzw. Isopropanol, zu verstehen.

Die kosmetischen Mittel (M1) in dem Container (C1) können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels (M1), enthalten ist.

Das kosmetische Mittel (M1) in dem Container (C1) enthält als ersten wesentlichen Bestandteil eine Verbindung, ausgewählt aus der Gruppe von Oxidationsfarbstoffvorprodukten (OFV), direktziehenden Farbstoffe (DZ) sowie deren Mischungen.

In einer bevorzugten Ausführungsform enthalten kosmetische Mittel (M1) in dem Container (C1) mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die kosmetischen Mittel (M1) in dem Container (C1) daher mindestens ein Oxidationsfarbstoffvorprodukt vom Entwicklertyp. Es kann im Rahmen der vorliegenden Erfindung jedoch auch vorgesehen sein, dass die kosmetischen Mittel (M1) mindestens ein Oxidationsfarbstoffvorprodukt vom Kupplertyp enthalten. Besonders gute Ergebnisse in Bezug auf die Färbung keratinischer Fasern werden erhalten, wenn die kosmetischen Mittel (M1) mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetischen Mittel (M1) bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1), enthalten.

In einer weiteren bevorzugten Ausführungsform ist das kosmetischen Mittel (M1) in dem Container (C1) daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1), enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1 ,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das kosmetische Mittel (M1) in dem Container (C1) als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus

(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,

(B) o-Aminophenol und dessen Derivaten, wie 2-Amino-5-ethylphenol,

(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,

(D) o-Diaminobenzol und dessen Derivaten,

(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,

(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,

(G) Naphthalinderivaten, wie 1-Naphthol und 2-Methyl-1-naphthol,

(H) Morpholinderivaten, wie 6-Hydroxybenzomorpholin,

(I) Chinoxalinderivaten,

(J) Pyrazolderivaten, wie 1-Phenyl-3-methylpyrazol-5-on,

(K) Indolderivaten, wie 6-Hydroxyindol,

(L) Pyrimidinderivaten oder

(M) Methylendioxybenzolderivaten, wie 1-(2'-Hydroxyethyl)-amino-3,4-methylendioxybenzol sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 4-Chlorresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die kosmetischen Mittel

(M1) in dem Container (C1) dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6-Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Um eine ausgewogene und subtile Nuancenausbildung zu erhalten, kann es im Rahmen der vorliegenden Erfindung auch vorgesehen sein, dass die kosmetischen Mittel (M1) in dem Container (C1) zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das kosmetische Mittel (M1) die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels (M1).

Als zweiten wesentlichen Bestandteil enthalten die kosmetischen Mittel (M1) in dem Container (C1) mindestens ein spezielles aminiertes Siliconpolymer. Der Zusatz dieses Siliconpolymers führt zu einer verbesserten Pflege, insbesondere Nasskämmbarkeit, und/oder zu einer verbesserten Buntheit von Farbnuancen.

Erfindungsgemäß enthält das kosmetische Mittel (M1) in dem Container (C1) mindestens ein aminiertes Siliconpolymer der Formel (III) in einer Gesamtmenge von 0,05 bis 1,0 Gew.-%, bezogen auf das Gesamtgewicht des kosmetischen Mittels (M1)

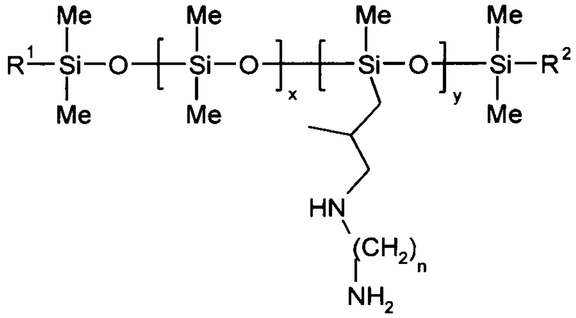

(III),

worin

$R^1$ und $R^2$, jeweils unabhängig voneinander, für eine Methylgruppe stehen,
x für ganze Zahlen von 49 bis 149, steht,
y für ganze Zahlen von 1 bis 10, steht, und
n 2 oder 3, steht.

Der Einsatz dieser speziellen aminierten Siliconpolymere resultiert in einer erhöhten Pflege der keratinischen Fasern nach der Farbveränderung und/oder in einer verbesserten Buntheit von Farbnuancen.

Bevorzugt weist das mindestens eine aminierte Siliconpolymer ein mittleres Molekulargewicht $M_w$ von 350 bis 350.000 Da, vorzugsweise von 500 bis 300.000 Da, bevorzugt von 700 bis 250.000 Da, insbesondere von 1.000 bis 200.000 Da, auf. Spezielle aminierte Siliconpolymere, welche das zuvor angeführte mittlere Molekulargewicht $M_w$ aufweisen, resultieren in einer besonders hohen Pflege keratinischer Fasern nach der Farbveränderung und/oder in einer verbesserten Buntheit von Farbnuancen. Das mittlere Molekulargewicht $M_w$ kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOFMS"; Am. Soc. Mass. Spectrom., 2003, 14, Seiten 195 bis 202).

Es hat sich als vorteilhaft erwiesen, wenn das mindestens eine aminierte Siliconpolymer eine Aminzahl oberhalb von 0,25 meq/g, vorzugsweise oberhalb von 0,3 meq/g, insbesondere oberhalb von 0,4 meq/g, aufweist.

Besonders bevorzugt ist es im Rahmen der vorliegenden Erfindung, wenn das mindestens eine aminierte Siliconpolymer eine Aminzahl von 0,25 bis 5 meq/g, vorzugsweise von 0,3 bis 4,5 meq/g, bevorzugt von 0,4 bis 4,0 meq/g, weiter bevorzugt von 0,5 bis 3,0 meq/g, insbesondere von 0,5 bis 1,5 meq/g, aufweist. Die Aminzahl steht dabei für die Milli-Äquivalente Amin pro Gramm des aminofunktionellen Silikons. Sie kann durch Titration ermittelt und auch in der Einheit mg KOH/g angegeben werden. Der Einsatz von aminierten Siliconpolymeren mit den zuvor genannten Aminzahlen resultiert in einer besonders hohen Pflege keratinischer Fasern, welche mit aus der erfindungsgemäßen Verpackungseinheit hergestellten oxidativen Färbemitteln gefärbt wurden. Weiterhin führt der Einsatz von Siliconpoylmeren mit Aminzahlen in den zuvor genannten Bereichen in den kosmetischen Mitteln (M1) der erfindungsgemäßen Verpackungseinheit zu einer verbesserten Buntheit von Farbnuancen.

Das mindestens eine aminierte Siliconpolymer ist in dem kosmetischen Mittel (M1) in dem Container (C1) in einer Gesamtmenge von 0,05 bis 1,0 Gew.-%, bezogen auf das Gesamtgewicht des kosmetischen Mittels (M1), enthalten. Der Einsatz der zuvor genannten Gesamtmenge des speziellen aminierten Siliconpolymers führt zu einer erhöhten Pflege der keratinischen Fasern und/oder zu einer verbesserten Buntheit von Farbnuancen.

Es hat sich gezeigt, dass ein Zusatz an cyclischen aminierten Siliconpolymeren im Rahmen der vorliegenden Erfindung besonders vorteilhaft ist. Erfindungsgemässe kosmetische Mittel (M1) enthalten daher zusätzlich ein cyclisches aminiertes Siliconpolymer in einer Gesamtmenge 0,015 bis 0,3 Gew.-%, bezogen auf das Gesamtgewicht des kosmetischen Mittels (M1), wobei das cyclische aminierte Siliconpolymer die Formel (IV) aufweist

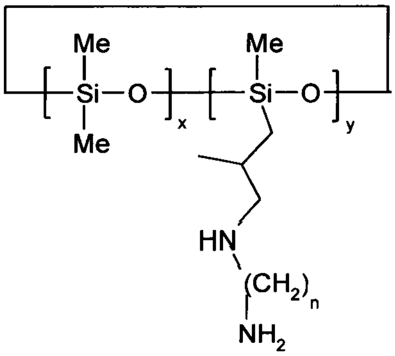

(IV),

worin

x für ganze Zahlen von 49 bis 149, steht, und
y für ganze Zahlen von 1 bis 10, steht.

Durch den Zusatz von aminierten cyclischen Siliconpolymeren kann die Pflegewirkung und/oder die Buntheit von Farbnuancen der aus der erfindungsgemäßen Verpackungseinheit hergestellten oxidativen Färbemittel weitergehend gesteigert werden.

Zudem hat sich der Zusatz von Dimethylcyclosiloxanen als vorteilhaft erweisen. Es ist daher im Rahmen der vorliegenden Erfindung bevorzugt, wenn die kosmetischen Mittel (M1) zusätzlich Dimethylcyclosiloxan in einer Gesamtmenge von weniger als 1 Gew.-%, bezogen auf das Gesamtgewicht des kosmetischen Mittels (M1), enthalten, wobei das Dimethylcyclosiloxan die Formel (V) aufweist

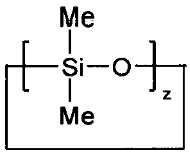

(V),

worin
z für ganze Zahlen von 2 bis 8, vorzugsweise von 2 bis 6, insbesondere für 3, 4, 5 oder 6, steht. Der Zusatz derartiger Dimethylcyclosiloxane zu den kosmetischen Mitteln (M1) führt zu einer weitergehenden Verbesserung der Pflegeleistung und/oder Verbesserung der Buntheit von Farbnuancen.

Die kosmetischen Mittel (M1) in dem Container (C1) können weitere Wirk- und Zusatzstoff enthalten. Es ist daher im Rahmen der vorliegenden Erfindung bevorzugt, wenn das kosmetische Mittel (M1) zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen;
(iii) Tensiden, insbesondere amphoteren Tensiden; (iv) Alkalisierungsmitteln; (v) Ölen; sowie (vi) deren Mischungen, enthält.

Bevorzugt werden die kosmetischen Mittel (M1) in dem Container (C1) als fließfähige Zubereitungen formuliert. Dabei sollten die kosmetischen Mittel (M1) so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die kosmetischen Mittel (M1) mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels (M1), enthalten.

Es hat sich im Rahmen dieser Ausführungsform als vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels (M1), enthalten ist.

Im Rahmen dieser Ausführungsform kann es weiterhin bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels (M1), enthalten ist.

Bevorzugt können die kosmetischen Mittel (M1) in dem Container (C1) weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten $C_{12}$ - $C_{30}$-Carbonsäuren, welche hydroxyliert sein können, insbesondere diejenigen mit Palmitin- und Stearinsäure, die Sorbitanmono-, -di- oder -triester von linearen gesättigten $C_{12}$ - $C_{30}$-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten $C_{12}$ - $C_{30}$-Carbonsäuren, welche hydroxyliert sein können.

Im Rahmen der vorliegenden Erfindung kann es vorgesehen sein, dass die kosmetischen Mittel (M1) in dem Container (C1) mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels (M1), enthalten.

Der Einsatz der zuvor angeführten Alkohole, Partialester und Polypartialester in den kosmetischen Mitteln (M1) kann insbesondere dann bevorzugt sein, wenn die kosmetischen Mittel (M1) in Form einer Öl-in-Wasser-Emulsion vorliegen.

Weiterhin kann es erfindungsgemäß vorgesehen sein, dass die kosmetischen Mittel (M1) in dem Container (C1) mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die kosmetischen Mittel (M1) mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels (M1). Als amphotere bzw. zwitterionische Tenside werden solche oberflächenaktiven Verbindungen bezeichnet, welche mindestens eine quartäre Ammoniumgruppe und mindestens eine $-COO^{(-)}$- oder-$SO3^{(-)}$-Gruppe aufweisen.

Als amphotere Tenside sind im Rahmen der vorliegenden Erfindung die nachfolgend genannten Verbindungen besonders bevorzugt:

- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die kosmetischen Mittel (M1) als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels (M1).

Weiterhin kann es vorgesehen sein, dass die kosmetischen Mittel (M1) mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels (M1), enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das kosmetische Mittel (M1) daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel (M1) im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels (M1), enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels (M1), enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem $C_2$-$C_6$-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte kosmetische Mittel (M1) enthalten eine Mischung aus Monoethanolamin und 2-Amino-2-methylpropan-1 ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels (M1), enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte kosmetische Mittel (M1) enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels (M1).

Erfindungsgemäß bevorzugt ist das Alkalisierungsmittel ausgewählt aus der Gruppe von Natriumhydroxid, Kaliumhydroxid, Ammoniak, Monoethanolamin und 2-Amino-2-methylpropan, vorzugsweise Monoethanolamin, und ist in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels (M1), enthalten.

In einer besonders bevorzugten Ausführungsform enthalten die kosmetischen Mittel (M1) als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels (M1).

Bevorzugt beträgt der pH-Wert der kosmetischen Mittel (M1), gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die kosmetischen Mittel (M1) mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in

einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels (M1), enthalten. Durch den Einsatz mindestens eines zuvor genannten Öls kann der Pflegeeffekt der aminierten Siliconpolymere weitergehend gesteigert werden.

Besonders bevorzugt enthalten die kosmetischen Mittel (M1) Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels (M1).

Gemäß einer besonders bevorzugten Ausführungsform der vorliegenden Erfindung enthalten die als Öl-in-Wasser-Emulsion vorliegenden kosmetischen Mittel (M1) in dem Container (C1) - bezogen auf das Gesamtgewicht der kosmetischen Mittel (M1) -

- Octyldodecanol in einer Gesamtmenge von 2,0 bis 20 Gew.-%, insbesondere von 5,0 bis 12 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Der Container (C2) der erfindungsgemäßen Verpackungseinheit enthält eine Oxidationsmittelzubereitung (M2), welche mindestens ein Oxidationsmittel enthält.

Die Oxidationsmittel im Rahmen der vorliegenden Erfindung sind von Luftsauerstoff verschieden. Als Oxidationsmittel in der Oxidationsmittelzubereitung (M2) können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass als Oxidationsmittel Wasserstoffperoxid in einer Gesamtmenge von 0,5 bis 20 Gew.-%, vorzugsweise von 2,0 bis 15 Gew.-%, bevorzugt von 3,0 bis 12 Gew.-%, besonders bevorzugt von 4,0 bis 10 Gew.-%, insbesondere von 5,5 bis 9,0 Gew.-%, bezogen auf das Gesamtgewicht der Oxidationsmittelzubereitung (M2), enthalten ist. Die Berechnung der Gesamtmenge bezieht sich hierbei auf 100 %-iges $H_2O_2$.

Die Oxidationsmittelzubereitungen (M2) in dem Container (C2) können weiterhin Wasser in einer Gesamtmenge von 40 bis 98 Gew.-%, insbesondere von 65 bis 85 Gew.-%, bezogen auf das Gesamtgewicht der Oxidationsmittelzubereitung (M2), enthalten.

Gemäß einer bevorzugten Ausführungsform der vorliegenden Erfindung enthalten die Oxidationsmittelzubereitungen (M2) weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung (M2). Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen (M2) mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung (M2).

Im Rahmen der vorliegenden Erfindung kann es darüber hinaus auch vorgesehen sein, dass die Oxidationsmittelzubereitungen (M2) mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung (M2), enthalten.

Gemäß einer besonders bevorzugten Ausführungsform der vorliegenden Erfindung enthalten die Oxidationsmittelzubereitungen (M2) in dem Container (C2) - bezogen auf das Gesamtgewicht der Oxidationsmittelzubereitungen (M2) -

- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen (M2) enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon. Die Oxidationsmittelzubereitungen weisen bevorzugt einen pH-Wert im Bereich von 2 bis 5, insbesondere von 3 bis 4, auf.

Zur Herstellung von oxidativen Färbezusammensetzungen aus der erfindungsgemäßen Verpackungseinheit (Kit-of-parts) wird das kosmetische Mittel (M1) in dem Container (C1) mit der Oxidationsmittelzubereitung (M2) in dem Container (C2) oder *vice versa* vermischt.

Weiterhin kann es erfindungsgemäß besonders vorteilhaft sein, wenn die erfindungsgemäße Verpackungseinheit mindestens ein weiteres Haarbehandlungsmittel, insbesondere eine Konditioniermittelzubereitung, in einem zusätzlichen Container enthält. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Ein weiterer Gegenstand der vorliegenden Erfindung ist ein Verfahren zur Färbung, insbesondere Aufhellung, keratinischer Fasern, wobei das Verfahren die folgenden Verfahrensschritte umfasst:

a) Bereitstellen einer erfindungsgemäßen Verpackungseinheit (Kit-of-parts),

b) Vermischen des in der erfindungsgemäßen Verpackungseinheit befindlichen kosmetischen Mittels (M1) und der Oxidationsmittelzubereitung (M2),

c) Applizieren der in Schritt c) erhaltenen Mischung auf die keratinischen Fasern und Belassen dieser Mischung auf den keratinischen Fasern für eine Zeit von 10 bis 60 Minuten, bevorzugt von 20 bis 45 Minuten, bei Raumtemperatur und/oder bei mindestens 30 °C,

d) Spülen der keratinischen Fasern mit Wasser und/oder einer reinigenden Zusammensetzung für 1 bis 5 Minuten, und

e) gegebenenfalls Applizieren eines Nachbehandlungsmittels auf die keratinischen Fasern und Spülen nach einer Zeit von 1 bis 10 Minuten.

Das erfindungsgemäße Verfahren zur Färbung keratinischer Fasern unter Verwendung eines speziellen aminierten Siliconpolymers resultiert in einer verbesserten Pflege gefärbter keratinischer Fasern und/oder einer verbesserten Buntheit von Farbnuancen.

Unter Raumtemperatur ist dabei im Rahmen der vorliegenden Erfindung die Umgebungstemperatur zu verstehen, welche ohne Einwirkung von externer Wärme vorherrscht und bevorzugt von 10 bis 39 °C beträgt. Die Wirkung der Färbezusammensetzung kann durch externe Wärmezufuhr, beispielsweise mittels einer Wärmehaube, verstärkt werden. Die bevorzugte Einwirkdauer der Färbezusammensetzung auf die keratinischen Fasern beträgt 10 bis 60 min, bevorzugt 20 bis 45 min. Nach Beendigung der Einwirkdauer wird das verbliebene Färbemittel aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen. Gegebenenfalls wird der Vorgang mit einem weiteren Mittel wiederholt. Nach dem Auswaschen werden die keratinischen Fasern gegebenenfalls mit einem Nachbehandlungsmittel, beispielsweise einem Konditioniermittel, gespült und mit einem Handtuch oder einem Heißluftgebläse

getrocknet. Die Auftragung der Färbezusammensetzung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel mit einer Applikationshilfe auf die keratinischen Fasern aufzutragen.

Besonders bevorzugte erfindungsgemäße Verfahren sind dadurch gekennzeichnet, dass die Verfahren in einer verbesserten Pflege und/oder Buntheit von Farbnuancen resultieren. Durch die Verwendung mindestens eines speziellen aminierten Siliconpolymers ist die aus dem erfindungsgemäßen Verfahren resultierende Pflege und/oder Buntheit von Farbnuancen größer als die Pflege und/oder Buntheit von Farbnuancen, welche in Abwesenheit des im Rahmen der vorliegenden Erfindung eingesetzten aminierten Siliconpolymers b) erreicht werden kann.

Bezüglich der im Rahmen des erfindungsgemäßen Verfahrens verwendeten kosmetischen Mittel (M1) und der Oxidationsmittelzubereitung (M2) sowie weiterer bevorzugter Ausführungsformen des erfindungsgemäßen Verfahrens gilt mutatis mutandis das zu der erfindungsgemäßen Verpackungseinheit Gesagte.

Schließlich ist ein weiterer Gegenstand der vorliegenden Erfindung die Verwendung einer erfindungsgemäßen Verpackungseinheit zur Herstellung eines kosmetischen Mittels zur Farbveränderung keratinischer Fasern mit erhöhter Pflege der keratinischen Fasern und/oder verbesserter Buntheit von Farbnuancen. Der Einsatz eines speziellen aminierten Siliconpolymers resultiert in einer erhöhten Pflege gefärbter keratinischer Fasern und/oder in einer verbesserten Buntheit von Farbnuancen.

Bezüglich weiterer bevorzugter Ausführungsformen der erfindungsgemäßen Verwendung gilt mutatis mutandis das zu der erfindungsgemäßen Verpackungseinheit sowie zu dem erfindungsgemäßen Verfahren Gesagte.

Die nachfolgenden Beispiele sollen bevorzugte Ausführungsformen der Erfindung erläutern.

Beispiele:

1. Rezepturen

Zusammensetzungen der eingesetzten kosmetischen Mittel (M1) (Öl-in-Wasser-Emulsionen, alle Mengen in Gew.-%). Das in den nachfolgenden Formulierungen verwendete aminierte Siliconpolymer ist bevorzugt ein Siliconpolymer der Formel (III) mit n = 2 oder 3 und einem mittleren Molekulargewicht $M_w$ von 1.000 bis 200.000 Da.

| Rohstoff | V1 | E1* | E2* |
|---|---|---|---|
| Xanthan Gum | 0,05 | 0,05 | 0,05 |
| 2-Octyldodecanol | 2,3 | 2,3 | 2,3 |
| Lanette N [a)] | 14 | 14 | 14 |
| Cetearyl Alkohol | 3,9 | 3,9 | 3,9 |
| Glycerinmonostearat | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 4,5 | 4,5 | 4,5 |
| 2-Amino-2-methylpropanol | 0,1 | 0,1 | 0,1 |
| Natriumsulfit, wasserfrei | 0,2 | 0,2 | 0,2 |
| Caramelsirup, 75%ig | 0,1 | 0,1 | 0,1 |
| Traubenkernöl | 1,0 | 1,0 | 1,0 |
| p-Toluylendiaminsulfat | 0,03 | 0,03 | 0,03 |
| 4-Amino-3-methylphenol | 0,3 | 0,3 | 0,3 |
| 1,3-Benzoldiol | 0,04 | 0,04 | 0,04 |
| 1-Naphthol | 0,09 | 0,09 | 0,09 |
| 5-Amino-2-methylphenol | 0,2 | 0,2 | 0,2 |
| 2-Amino-6-chloro-4-nitrophenol | 0,2 | 0,2 | 0,2 |
| Aminiertes Siliconpolymer ** | - | 0,97 | 1,9 |

(fortgesetzt)

| Rohstoff | V1 | E1* | E2* |
|---|---|---|---|
| Wasser, vollentsalzt | ad 100,00 | ad 100,00 | ad 100,00 |
| * erfindungsgemäß<br>** Aktivsubstanz<br>a) INCI-Bezeichnung: Cetearyl alcohol, Sodium cetearyl sulfate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur ohne aminiertes Siliconpolymer. Die Rezepturen E1 und E2 sind erfindungsgemäße Beispiele.

Oxidationsmittelzubereitung O1 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,22 |
| 1-Hydroxyethan-1,1-diphosphonsäure 60% | 0,25 |
| Emulgade F b) | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 11,2 |
| Wasser vollentsalzt | ad 100 |
| b) INCI-Bezeichnung: Cetearyl alcohol, PEG-40 Castor oil, Sodium cetearyl sulfat (BASF) | |

2. Verbesserte Pflege durch Zusatz mindestens eines speziellen aminierten Siliconpolymers

Zur Herstellung der oxidativen Färbemittel zur Bestimmung der Pflege wurden die kosmetischen Mittel V1, E1 und E2 jeweils im Gewichtsverhältnis 1:1 mit der obigen Oxidationsmittelzubereitung O1 vermischt.

12 Strähnen von natürlich hellbraunem europäischen Haar (IHIP (New York), lot# 03/2012, N104, length 15 cm, weight 1 g) wurden mit einer wässrigen Natrium-Laurylethersulfat-Lösung (3 % Aktivsubstanzgehalt in der Lösung) gewaschen. Die Strähnen wurden an der Luft getrocknet und für 24 h bei 25°C und 25% relativer Luftfeuchtigkeit gelagert. Nach Einweichen dieser Strähnen für 5 Minuten in Wasser wurde deren Nasskämmbarkeit bestimmt (Referenzwert).

Für die Färbungen wurden jeweils 12 Strähnen von natürlich europäischen Haar (IHIP (New York), lot # 03/2012, N104, length 15 cm, weight 1 g) je oxidativem Färbemittel verwendet. Hierfür wurden jeweils 4 g der zuvor hergestellten oxidativen Färbemittel pro 1 g Haarsträhne appliziert. Nachdem die Strähnen für 30 min bei 32 °C gefärbt wurden, wurden sie für 2 min mit Wasser gespült und an der Luft getrocknet.

Die Messung der Nasskämmbarkeit wurde wie folgt durchgeführt:
Vor der Messung wurde jede Strähne unter Kämmen mit einem harten Gummikamm mit feinen Zähnen (Firma Hercules Sägemann, Hamburg Germany) für 2 Sekunden mit Wasser befeuchtet. Nachdem 3 Kämmvorgänge durchgeführt wurden, wird die Kämmkraft während weiteren 10 Kämmvorgängen gemessen, wobei die jeweilige Haarsträhne während des Kämmvorgangs langsam rotiert. Die erhaltenen Messwerte werden unter Verwendung der folgenden in der Software Statistica 10.0 (StatSoft Inc., USA) eingebetteten statistischen Tests verglichen:

- Shapiro-Wilks Test (Test für Normalverteilung)
- Ausreißertest nach Grubbs

- Bartlett-Test (Test auf Homoskedastizität von Varianzen)
- Univarianter Signifikanztest
- Newman-Keuls Test (Bestimmung von signifikanten Unterschieden)
- Unequal N HSD Test (Test auf Mehrfachvergleiche).

Die Änderung der Kämmkraft dK in Prozent kann mit Hilfe der Formel dK = $[(K_0-K_i)/K_0]*100$ berechnet werden. $K_0$ ist hierbei der Mittelwert der Kämmkraft für die ungefärbten Haarsträhnen und $K_i$ der Mittelwert für die mit dem jeweiligen oxidativen Färbemittel behandelten Haarsträhnen.

Die Pflege der Haarsträhnen ist umso höher, je geringer die aufgewendete Kämmkraft und damit je höher die Änderung der Kämmkraft ist. In Tabelle 2 sind die dK-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche mindestens ein spezielles aminiertes Siliconpolymer in einer Gesamtmenge von 0,97 Gew.-% bzw. 1,9 Gew.-% enthalten, weisen im Vergleich zu den Ausfärbungen ohne aminiertes Siliconpolymer (V1) eine höhere Änderung der Kämmkraft und somit eine erhöhte Pflege auf.

| Oxidatives Färbemittel | dK [%] |
|---|---|
| V1 + O1 (1:1) | 36 |
| E1 + O1 (1:1) | 48 |
| E2 + O1 (1:1) | 54 |

3. Verbesserte Buntheit von Farbnuancen durch Zusatz mindestens eines speziellen aminierten Siliconpolymers

Zur Herstellung der oxidativen Färbemittel für die Bestimmung der Buntheit und Farbtiefe wurden die kosmetischen Mittel V1 sowie E1 und E2 jeweils im Gewichtsverhältnis 1:1 mit der obigen Oxidationsmittelzubereitung O1 vermischt.

Die auf diese Weise hergestellten oxidativen Färbemittel wurden jeweils in definierter Menge (4 g oxidatives Färbemittel pro 1 g Yakhaar) auf Yakhaar-Strähnen aufgetragen (jeweils 12 Strähnen pro oxidativem Färbemittel) und verblieben für eine Einwirkdauer von 30 Minuten bei 32 °C auf den Haarsträhnen. Anschließend wurden die verbliebenen Mittel jeweils 2 Minuten lang mit lauwarmem Wasser aus den Haarsträhnen ausgespült, die Strähnen zunächst mit einem Handtuch getrocknet und anschließend trocken geföhnt (Ausfärbungen: leuchtendes Rot).

Alle Strähnen wurden mit einem Farbmessgerät der Firma Datacolor, Typ Spectraflash 450, vermessen. Der für die Beurteilung der Buntheit herangezogene Wert dC ergibt sich aus den an der jeweiligen Strähne gemessenen a*b-Farbmesswerten wie folgt:

$$dC = [(a_i-a_0)^2+(b_i-b_0)^2]^{1/2}$$

ao und bo sind hierbei jeweils die Mittelwerte der aus den 12 Messungen ermittelten Farbmesswerte der unbehandelten Yakhaar-Strähnen, während $a_i$ und $b_i$ die Mittelwerte der Farbmesswerte nach der oxidativen Färbung der Haarsträhnen mit dem jeweiligen oxidativen Färbemittel darstellen.

Ein Indikator für die Buntheit der Ausfärbungen ist das Chroma. Je höher der dC-Wert ist, desto höher ist die Buntheit der Nuance. In nachfolgender Tabelle sind die dC-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche mindestens ein spezielles aminiertes Siliconpolymer in einer Gesamtmenge von 0,97 Gew.-% bzw. 1,9 Gew.-% enthalten, weisen im Vergleich zu den Ausfärbungen ohne aminiertes Siliconpolymer (V1) eine verbesserte Buntheit auf.

| Oxidatives Färbemittel | dC |
|---|---|
| V1 + O1 (1:1) | 38,74 |
| E1 + O1 (1:1) | 41,04 |
| E2 + O1 (1:1) | 39,50 |

**Patentansprüche**

**1.** Verpackungseinheit (Kit-of-Parts) zur Färbung keratinischer Fasern, umfassend - getrennt voneinander konfektioniert -

a) mindestens einen Container (C1), enthaltend ein kosmetisches Mittel (M1), welches mindestens eine Verbindung, ausgewählt aus der Gruppe von Oxidationsfarbstoffvorprodukten, direktziehenden Farbstoffen sowie deren Mischungen, und mindestens ein aminiertes Siliconpolymer, enthaltend mindestens eine Struktureinheit der Formel (I) und mindestens eine Struktureinheit der Formel (II)

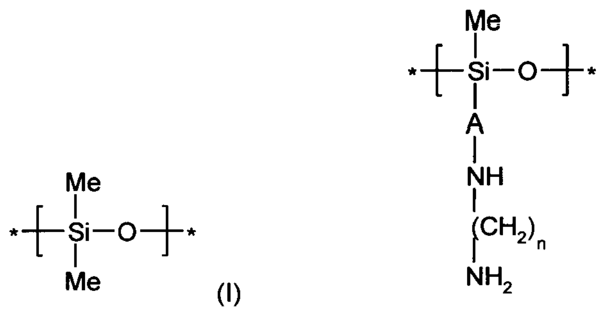

(I) (II),

worin

A für eine lineare oder verzweigte $C_4$-$C_8$-Alkylgruppe steht, und
n für ganze Zahlen von 1 bis 4 steht, enthält,
wobei das kosmetische Mittel (M1) in dem Container (C1) als aminiertes Siliconpolymer vorliegt, enhaltend mindestens eine Struktureinheit der Formel (I) und mindestens eine Struktureinheit der Formel (II), darunter mindestens ein aminiertes Siliconpolymer der Formel (III) in einer Gesamtmenge von 0,05 bis 1,0 Gew.-%, bezogen auf das Gesamtgewicht des kosmetischen Mittels (M1), enthält

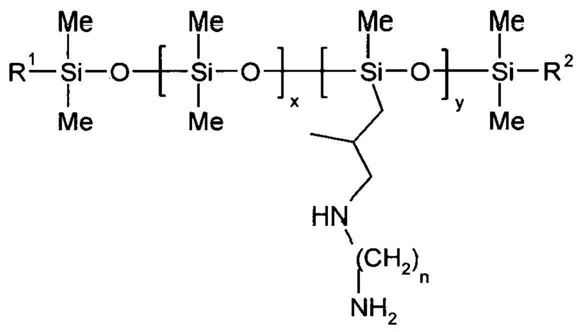

(III),

worin

$R^1$ und $R^2$, jeweils unabhängig voneinander, für eine Methylgruppe stehen,
x für ganze Zahlen von 49 bis 149, steht,
y für ganze Zahlen von 1 bis 10, steht, und
n für 2 oder 3, steht,
wobei das kosmetische Mittel (M1) zusätzlich ein cyclisches aminiertes Siliconpolymer in einer Gesamtmenge von 0,015 bis 0,3 Gew.-%, bezogen auf das Gesamtgewicht des kosmetischen Mittels (M1), enthält, wobei das cyclische aminierte Siliconpolymer die Formel (IV) aufweist

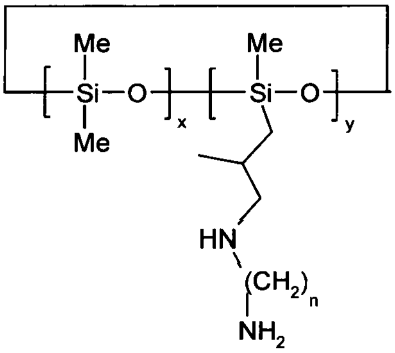

(IV),

worin

x für ganze Zahlen von 49 bis 149, steht, und
y für ganze Zahlen von 1 bis 10, steht

b) mindestens einen Container (C2), enthaltend eine Oxidationsmittelzubereitung (M2), welche in einem kosmetisch verträglichen Träger mindestens ein Oxidationsmittel in einer Gesamtmenge von 0,5 bis 15 Gew.-%, vorzugsweise von 0,75 bis 10 Gew.-%, bevorzugt von 1 bis 7,5 Gew.-%, besonders bevorzugt von 1,25 bis 7 Gew.-%, insbesondere von 1,5 bis 6,0 Gew.-%, bezogen auf das Gesamtgewicht der Oxidationsmittelzubereitung, enthält.

2. Verpackungseinheit nach Anspruch 1, **dadurch gekennzeichnet, dass** das mindestens eine aminierte Siliconpolymer der Formel III ein mittleres Molekulargewicht $M_w$ von 350 bis 350.000 Da, vorzugsweise von 500 bis 300.000 Da, bevorzugt von 700 bis 250.000 Da, insbesondere von 1.000 bis 200.000 Da, aufweist.

3. Verpackungseinheit nach einem der vorangehenden Ansprüche, **dadurch gekennzeichnet, dass** das mindestens eine aminierte Siliconpolymer der Formel III eine Aminzahl von 0,25 bis 5 meq/g, vorzugsweise von 0,3 bis 4,5 meq/g, bevorzugt von 0,4 bis 4,0 meq/g, weiter bevorzugt von 0,5 bis 3,0 meq/g, insbesondere von 0,5 bis 1,5 meq/g, aufweist.

4. Verpackungseinheit nach einem der vorangehenden Ansprüche, **dadurch gekennzeichnet, dass** das kosmetische Mittel (M1) zusätzlich Dimethylcyclosiloxan in einer Gesamtmenge von weniger als 1 Gew.-%, bezogen auf das Gesamtgewicht des kosmetischen Mittels (M1), enthält, wobei das Dimethylcyclosiloxan die Formel (V) aufweist

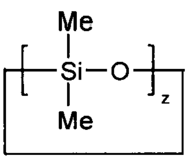

(V),

worin z für ganze Zahlen 3, 4, 5 oder 6, steht.

5. Verpackungseinheit nach einem der vorangehenden Ansprüche, **dadurch gekennzeichnet, dass** das kosmetische Mittel (M1) zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

6. Verpackungseinheit nach Anspruch 5, **dadurch gekennzeichnet, dass** als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels (M1), enthalten ist.

7. Verpackungseinheit nach einem der vorangehenden Ansprüche, **dadurch gekennzeichnet, dass** das Oxidationsmittel ausgewählt ist aus der Gruppe von Persulfaten, Chloriten, Wasserstoffperoxid und Anlagerungsprodukten

von Wasserstoffperoxid an Harnstoff, Melamin sowie Natriumborat, insbesondere Wasserstoffperoxid.

**8.** Verfahren zum Färben von keratinischen Fasern, wobei das Verfahren die folgenden Verfahrensschritte umfasst:

a. Bereitstellen einer Verpackungseinheit (Kit-of-parts) nach einem der Ansprüche 1 bis 7,
b. Vermischen des in der Verpackungseinheit nach einem der Ansprüche 1 bis 11 befindlichen kosmetischen Mittels (M1) und der Oxidationsmittelzubereitung (M2),
c. Applizieren der in Schritt c) erhaltenen Mischung auf die keratinischen Fasern und Belassen dieser Mischung auf den keratinischen Fasern für eine Zeit von 10 bis 60 Minuten, bevorzugt von 20 bis 45 Minuten, bei Raumtemperatur und/oder bei mindestens 30 °C,
d. Spülen der keratinischen Fasern mit Wasser und/oder einer reinigenden Zusammensetzung für 1 bis 5 Minuten, und
e. gegebenenfalls Applizieren eines Nachbehandlungsmittels auf die keratinischen Fasern und Spülen nach einer Zeit von 1 bis 10 Minuten.

**Claims**

**1.** A packaging unit (kit-of-parts) for dyeing keratin fibers, comprising - packaged separately from one another -

a) at least one container (C1) containing a cosmetic agent (M1) that contains at least one compound selected from the group of oxidation dye precursors, direct dyes and mixtures thereof, and at least one aminated silicone polymer containing at least one structural unit of formula (I) and at least one structural unit of formula (II)

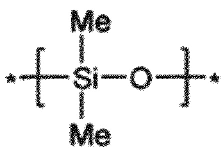
(I)

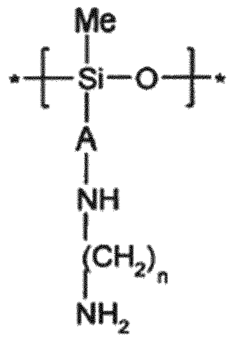
(II),

in which

A represents a linear or branched C4-C8 alkyl group, and
n represents integers from 1 to 4,
wherein the cosmetic agent (M1) is present in the container (C1) as an aminated silicone polymer containing at least one structural unit of formula (I) and at least one structural unit of formula (II), including at least one aminated silicone polymer of formula (III) in a total of amount from 0.05 to 1.0 wt.%, based on the total weight of the cosmetic agent (M1),

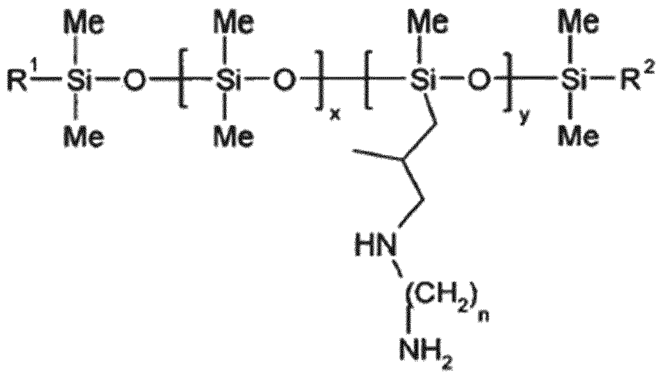

(III),

in which

R1 and R2 each independently represent a methyl group,
x represents integers from 49 to 149,
y represents integers from 1 to 10, and
n represents 2 or 3,
wherein the cosmetic agent (M1) additionally contains a cyclic aminated silicone polymer in a total amount of from 0.015 to 0.3 wt.% based on the total weight of the cosmetic agent (M1), wherein the cyclic aminated silicone polymer has the formula (IV)

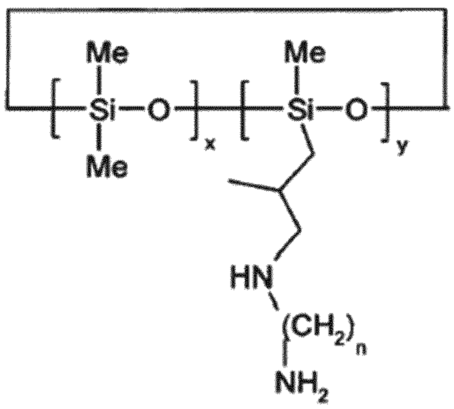

(IV),

in which

x represents integers from 49 to 149, and
y represents integers from 1 to 10,

b) at least one container (C2) containing an oxidizing agent preparation (M2) which, in a cosmetically acceptable carrier, contains at least one oxidizing agent in a total amount of from 0.5 to 15 wt.%, preferably from 0.75 to 10 wt.%, more preferably from 1 to 7.5 wt.%, particularly preferably from 1.25 to 7 wt.%, in particular from 1.5 to 6.0 wt.%, based on the total weight of the oxidizing agent preparation.

**2.** The packaging unit according to claim 1, **characterized in that** the at least one aminated silicone polymer of formula III has an average molecular weight Mw of from 350 to 350,000 Da, preferably from 500 to 300,000 Da, more preferably from 700 to 250,000 Da, in particular from 1,000 to 200,000 Da.

**3.** The packaging unit according to one of the preceding claims, **characterized in that** the at least one aminated silicone polymer of formula III has an amine number of from 0.25 to 5 meq/g, preferably from 0.3 to 4.5 meq/g, more preferably from 0.4 to 4.0 meq/g, even more preferably from 0.5 to 3.0 meq/g, in particular from 0.5 to 1.5 meq/g.

4. The packaging unit according to one of the preceding claims, **characterized in that** the cosmetic agent (M1) additionally contains dimethylcyclosiloxane in a total amount of less than 1 wt.% based on the total weight of the cosmetic agent (M1), the dimethylcyclosiloxane having formula (V)

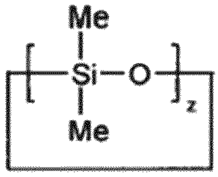

(V),

in which
z represents integers 3, 4, 5 or 6.

5. The packaging unit according to one of the preceding claims, **characterized in that** the cosmetic agent (M1) additionally contains at least one further compound selected from the group of (i) thickeners; (ii) linear or branched, saturated or unsaturated alcohols having 8 to 20 carbon atoms; (iii) surfactants, in particular amphoteric surfactants; (iv) alkalizing agents; (v) oils; and (vi) mixtures thereof.

6. The packaging unit according to claim 5, **characterized in that**, as an alkalizing agent, a mixture of at least two different alkanolamines, in particular monoethanolamine and 2-amino-2-methylpropan-1-ol, is contained in a total amount of from 0.05 to 15 wt.%, preferably from 0.5 to 10 wt.%, in particular from 3.5 to 7.5 wt.%, based on the total weight of the cosmetic agent (M1).

7. The packaging unit according to one of the preceding claims, **characterized in that** the oxidizing agent is selected from the group of persulfates, chlorites, hydrogen peroxide and addition products of hydrogen peroxide to urea, melamine, and sodium borate, in particular hydrogen peroxide.

8. A method for oxidatively dyeing keratin fibers, wherein the method comprises the following method steps:

a. providing a packaging unit (kit-of-parts) according to one of claims 1 to 7,
b. mixing the cosmetic agent (M1) and the oxidizing agent preparation (M2) present in the packaging unit according to one of claims 1 to 11,
c. applying the mixture obtained in step c) to the keratin fibers and leaving this mixture on the keratin fibers for a period of from 10 to 60 minutes, preferably from 20 to 45 minutes, at room temperature and/or at at least 30 °C,
d. rinsing the keratin fibers with water and/or a cleansing composition for 1 to 5 minutes, and
e. optionally applying a post-treatment agent to the keratin fibers and rinsing after a period of from 1 to 10 minutes.

## Revendications

1. Unité d’emballage (kit de pièces) pour la coloration des fibres kératiniques, comprenant - assemblés séparément -

a) au moins un récipient (C1) contenant un agent cosmétique (M1) qui contient au moins un composé choisi dans le groupe constitué des précurseurs de colorants par oxydation, des colorants directs ainsi que de leurs mélanges, et au moins un polymère de silicone aminé contenant au moins un motif structural de formule (I) et au moins un motif structural de formule (II)

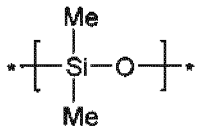 (I) 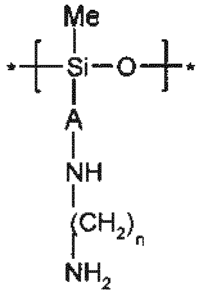 (II),

dans laquelle

A représente un groupe alkyle en $C_4$-$C_8$ linéaire ou ramifié, et
n représente des nombres entiers compris entre 1 et 4,
l'agent cosmétique (M1) étant présent dans le récipient (C1) sous la forme d'un polymère de silicone aminé, contenant au moins un motif structural de formule (I) et au moins un motif structural de formule (II), notamment au moins un polymère de silicone aminé de formule (III) en une quantité totale de 0,05 à 1,0 % en poids, par rapport au poids total de l'agent cosmétique (M1),

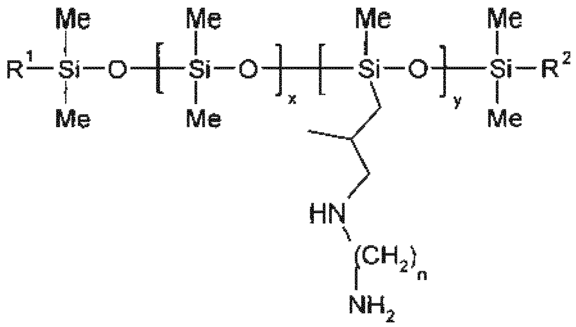

(III),

dans laquelle

$R^1$ et $R^2$, chacun indépendamment l'un de l'autre, représentent un groupe méthyle.
x représente des nombres entiers compris entre 49 et 149,
y représente des nombres entiers compris entre 1 et 10,
n représente 2 ou 3,
l'agent cosmétique (M1) contenant en outre un polymère de silicone aminé cyclique en une quantité totale de 0,015 à 0,3 % en poids par rapport au poids total de l'agent cosmétique (M1), ledit polymère de silicone aminé cyclique présentant la formule (IV)

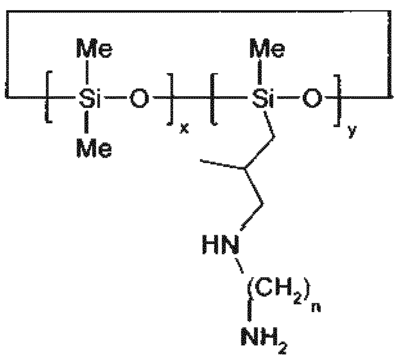

(IV),

dans laquelle

x représente des nombres entiers compris entre 49 et 149, et

y représente des nombres entiers compris entre 1 et 10,

b) au moins un récipient (C2) contenant une préparation d'agent oxydant (M2) qui contient, dans un support cosmétiquement acceptable, au moins un agent oxydant en une quantité totale de 0,5 à 15 % en poids, de préférence de 0,75 à 10 % en poids, de manière préférée de 1 à 7,5 % en poids, de manière particulièrement préférée de 1,25 à 7 % en poids, en particulier de 1,5 à 6,0 % en poids par rapport au poids total de la préparation d'agent oxydant.

**2.** Unité d'emballage selon la revendication 1, **caractérisée en ce qu'**au moins un polymère de silicone aminé de formule III présente un poids moléculaire moyen $M_w$ de entre 350 à 350 000 Da, de préférence de 500 à 300 000 Da, de manière préférée de 700 à 250 000 Da, en particulier de 1 000 à 200 000 Da.

**3.** Unité d'emballage selon l'une des revendications précédentes, **caractérisée** en cequ'au moins un polymère de silicone aminé de formule III présente un indice d'amine de 0,25 à 5 meq/g, de préférence de 0,3 à 4,5 meq/g, de manière préférée de 0,4 à 4,0 meq/g, de manière davatage préférée de 0,5 à 3,0 meq/g, en particulier de 0,5 à 1,5 meq/g.

**4.** Unité d'emballage selon l'une des revendications précédentes, **caractérisée en ce que** l'agent cosmétique (M1) contient en outre du diméthylcyclosiloxane en une quantité totale inférieure à 1 % en poids, par rapport au poids total de l'agent cosmétique (M1), le diméthylcyclosiloxane présentant la formule (V)

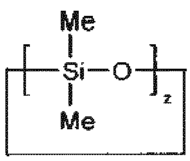
(V),

dans laquelle
z représente les nombres entiers 3, 4, 5 ou 6.

**5.** Unité d'emballage selon l'une des revendications précédentes, **caractérisée en ce que** l'agent cosmétique (M1) contient en outre au moins un autre composé choisi dans le groupe constitué (i) des épaississants ; (ii) des alcools linéaires ou ramifiés, saturés ou insaturés ayant de 8 à 20 atomes de carbone ; (iii) des tensioactifs, en particulier des tensioactifs amphotères ; (iv) des alcalinisants ; (v) des huiles ; ainsi que (vi) leurs mélanges.

**6.** Unité d'emballage selon la revendication 5, **caractérisée en ce qu'**un mélange d'au moins deux alcanolamines différentes, en particulier de monoéthanolamine et de 2-amino-2-méthylpropan-1-ol, est présent comme alcalinisant en une quantité totale de 0,05 à 15 % en poids, de préférence de 0,5 à 10 % en poids, en particulier de 3,5 à 7,5 % en poids, par rapport au poids total de l'agent cosmétique (M1).

**7.** Unité d'emballage selon l'une des revendications précédentes, **caractérisée en ce que** l'agent oxydant est choisi dans le groupe constitué des persulfates, des chlorites, du peroxyde d'hydrogène et des produits d'addition de peroxyde d'hydrogène en urée, en mélamine et en borate de sodium, en particulier en peroxyde d'hydrogène.

**8.** Procédé de coloration de fibres kératiniques, le procédé comprenant les étapes de procédé suivantes :

a. la fourniture d'une unité d'emballage (kit de pièces) selon l'une quelconque des revendications 1 à 7,
b. le mélange de l'agent cosmétique (M1) présent dans l'unité de conditionnement selon l'une des revendications 1 à 11 et de la préparation d'agent oxydant (M2),
c. l'application du mélange obtenu à l'étape c) sur les fibres kératiniques et le fait de laisser ce mélange sur les fibres kératiniques pendant une durée de 10 à 60 minutes, de préférence de 20 à 45 minutes, à température ambiante et/ou à au moins 30°°C,
d. le rinçage des fibres kératiniques avec de l'eau et/ou une composition détergente pendant 1 à 5 minutes, et
e. le cas échéant, l'application d'un agent de post-traitement sur les fibres kératiniques et le rinçage après une durée de 1 à 10 minutes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 2003152534 A1
- WO 03009822 A2
- US 2003229947 A1
- WO 0247632 A2
- FR 2945731 A1
- EP 1426038 A1
- WO 2012079915 A2

### In der Beschreibung aufgeführte Nicht-Patentliteratur

- **LIU X. M.** Comparative Studies of Poly(DimethylSiloxanes) Using Automated GPC-MALDI-TOF MS and On-Line GPC-ESI-TOFMS. *Am. Soc. Mass. Spectrom.,* 2003, vol. 14, 195-202